(19) Europäisches Patentamt
European Patent Office
Office européen des brevets

(11) Numéro de publication: **0 277 876 A2**

(12) # DEMANDE DE BREVET EUROPEEN

(21) Numéro de dépôt: **88400205.6**

(22) Date de dépôt: **29.01.88**

(51) Int. Cl.$^4$: **A 61 K 7/075**

(30) Priorité: **29.01.87 FR 8701060**

(43) Date de publication de la demande: **10.08.88 Bulletin 88/32**

(84) Etats contractants désignés: **AT BE CH DE ES FR GB GR IT LI LU NL SE**

(71) Demandeur: **LABORATOIRE LACHARTRE SOCIETE ANONYME:**
**104 Avenue du Général de Gaulle**
**F-92200 Neuilly sur Seine (FR)**

(72) Inventeur: **Scandel, Jean**
**1, Square Villaret de Joyeuse**
**F-75017 Paris (FR)**

(74) Mandataire: **L'Helgoualch, Jean**
**OFFICE PICARD 134 Boulevard de Clichy**
**F-75018 Paris (FR)**

(54) **Composition pour le lavage et le conditionnement de la chevelure en une seule application.**

(57) L'invention concerne une nouvelle composition pour le lavage et le conditionnement de la chevelure.

La composition est constituée par une solution aqueuse de pH comprise entre 5,5 et 6,5 environ, comprenant : a - 9 à 12% en poids environ d'au moins un surfactif anionique;

b - 1 à 3% en poids environ d'au moins un oxyde d'amine présentant un effet de conditionneur;

c - 0,2 à 1,5%.en poids environ d'au moins un polymère quaternaire cationique présentant un effet de conditionneur;

d - 0,7 à 2% en poids environ d'au moins un surfactif amphotère;

le reste de la composition comprenant des additifs connus.

Application au lavage et au conditionnement de la chevelure en une seule application.

EP 0 277 876 A2

**Description**

Composition pour le lavage et le conditionnement de la chevelure en une seule application.

La présente invention concerne une nouvelle composition pour le lavage et le conditionnement de la chevelure, et plus particulièrement une nouvelle composition procurant une action à la fois de shampooing et de conditionneur en une seule application.

On connait divers types de shampooings dont la composition est généralement à base de surfactifs, qui peuvent être anioniques, tels qu'un alkyl sulfate, un alkyl éther sulfate, un alkyl sulfonate, un alkyl sulfo succinate, ou encore un alkyl phosphate de métal alcalin, comportant 12 à 24 atomes de carbone dans la partie alkyle, ou amphotères tels qu'une N-alkyl amido bétaïne, ou cationiques tels qu'un acétate d'alkyl ammonium, ou encore non ioniques tels qu'un alcool gras oxyéthylèné. Ces shampooings sont utilisés normalement en petites quantités appliquées sur les cheveux par l'utilisateur qui, après addition d'eau et moussage, procède ensuite à un rinçage. Il est généralement nécessaire d'effectuer deux fois cette opération pour obtenir un résultat satisfaisant.

Ces surfactifs sont plutôt irritants pour le cuir chevelu; de plus, leur action détergente au cours du lavage a pour effet d'éliminer en partie le sebum des cheveux, ce qui a tendance à les rendre secs et difficiles à coiffer. Les shampooings usuels doivent donc comporter divers additifs pour compenser les effets secondaires des surfactifs et compléter leur action, et il est même nécessaire, lorsque l'on veut restaurer et améliorer le lustre et la souplesse des cheveux et leur facilité de coiffage, d'utiliser un conditionneur, appliqué après le shampooing. De plus, un rinçage est indispensable après les applications du shampooing et du conditionneur, c'est-à-dire qu'il faut procéder en deux étapes impliquant l'utilisation d'un shampooing suivie de celle d'un conditionneur, chaque application étant suivie d'un rinçage.

Les conditionneurs utilisés habituellement possèdent un pH relativement acide, généralement comprise entre 3 et 3,5. En outre, il est difficile de combiner un shampooing et un conditionneur dans une seule composition, car les propriétés d'une telle combinaison sont difficiles à prévoir, en raison des interactions des divers composants, et même lorsqu'il y a compatibilité chimique entre les divers composants, leurs propriétés s'avèrent habituellement antagonistes pour l'obtention simultanée du lavage et de la restauration de la chevelure.

Un shampooing-conditionneur est décrit dans le brevet FR-A-2.233.981, constitué essentiellement d'un mélange particulier de surfactifs amphotère, anionique et cationique, impliquant notamment l'utilisation d'un surfactif crypto-anionique. Un autre exemple de shampooing présenté comme possédant un effect conditionneur, et comportant un surfactif anionique, un surfactif amphotère ou non ionique, et un polymère cationique, est décrit dans le brevet FR-A-2.197.567; toutefois cette composition possède un pH basique, et ne peut pas être utilisée efficacement en une seule application. Le brevet US-A-3.761.418 décrit une composition détergente à base de surfactif anionique et d'un polymère cationique hydrosoluble, suceptible de s'appliquer à un shampooing traitant, par exemple antipelliculaire. L'utilisation d'un oxyde d'amine est envisagée dans le brevet FR-A-2.113.045, à titre de composant secondaire additionné à une composition d'un antimicrobien dermique à base de résine hydrosoluble et d'un surfactif. Enfin, une composition d'un copolymère à groupe ammonium quaternaire spécifique et d'un surfactif, pouvant contenir divers additifs usuels, est décrite dans Research Disclosure, n°233, p.298 (septembre 1983).

La présente invention a pour objet une nouvelle composition spécifique pour le lavage et le conditionnement des cheveux, évitant les inconvénients décrits ci-dessus, et procurant en une seule opération l'action de lavage du shampooing et l'action de restauration de la qualité des cheveux, du conditionneur.

La composition pour le le lavage et le conditionnement des cheveux agissant comme shampooing et comme conditionneur selon la présente invention se distingue en ce qu'elle est constituée par une solution aqueuse de pH comprise entre 5 et 7 environ, comprenant : a - 8 à 20% en poids environ d'au moins un surfactif anionique;

b - 1 à 3,5% en poids environ d'au moins un oxyde d'amine présentant un effet de conditionneur, comportant dans sa molécule au moins un groupe alkyle linéaire ou ramifié, ou une chaîne alkylène linéaire ou ramifiée, contenant au moins 14 atomes de carbone;

c - 0,2 à 3% en poids environ d'au moins un polymère quanternaire cationique présentant un effet de conditionneur;

le reste de la composition comprenant des additifs connus.

Bien entendu, la composition suivant l'invention peut encore contenir des ingrédients choisis parmi ceux couramment utilisés dans les shampooings et les conditionneurs, tels que des agents moussants, des épaississants, des silicones, des protéines, des solvants organiques appropriés, des parfums, des colorants, des anti-oxydants, etc. Tous ces additifs conventionnels sont choisis par l'homme du métier en fonction de leur compatibilité et des résultats recherchés.

Il est également préférable d'incorporer à la composition au moins un agent humectant et/ou un agent séquestrant, pour favoriser les propriétés d'application sur les cheveux.

De plus, l'incorporation d'au moins un surfactif amphotère en une quantité représentant au plus 6% en poids du poids total de la composition, et de préférence entre 0,7 et 2% en poids, peut s'avérer avantageuse, conformément à la présente invention.

Suivant une forme avantageuse de réalisation de l'invention, l'agent humectant est une urée, ou un dérivé, présent à raison de 1 à 5% en poids environ, et de préférence 2 à 4% en poids, par rapport au poids total de la composition. Un tel composé utilisé dans une telle quantité, permet de faciliter le pouvoir de mouillabilité de la composition. Bien que ses propriétés soient connues, l'urée est peu utilisée dans les produits capillaires, et son utilisation dans une composition combinant un shampooing et un conditionneur n'avait pas encore été envisagée.

L'agent séquestrant utilisable dans la composition de la présente invention peut être choisi parmi l'acide éthylène diamine tétracétique, l'acide nitrilotriacétique ou un polyphosphate. Suivant l'invention, on utilise de préférence l'acide nitrilotriacétique présent à raison de 0,2 à 2% en poids environ du poids total de la composition. Un tel composé permet d'améliorer les propriétés de moussage, d'application et de rinçage de la composition suivant l'invention. Suivant une forme préférentielle de réalisation, il est utilisé à raison de 0,25 à 1% en poids environ.

Comme indiqué ci-dessus, le pH de la composition suivant l'invention est compris entre 5 et 7 environ, c'est-à-dire dans un domaine de faible acidité, et de préférence entre 5,5 et 6,5. La valeur du pH dépend bien entendu des divers composants utilisés, mais elle doit être ajustée de manière usuelle, si nécessaire, pour être comprise dans le domaine précité. Il est important de noter que les oxydes d'amine classiques de la technique ne sont fortement substantifs qu'à des valeurs plus acides du pH, c'est-à-dire entre 3 et 4 environ, et qu'une augmentation du pH entraîne une diminution de leur cationicité et de leur substantivité. En corrélation, la composition de l'invention procure, à un pH légèrement acide, une excellente action de conditionneur sans effet substantif excessif, en plus d'une action de shampooing, et elle peut être utilisée avec la même fréquence qu'un shampooing classique.

Les surfactifs anioniques et amphotères utilisés dans la présente invention peuvent être choisis parmi les composés utilisés couramment dans les shampooings, et constituant une base lavante satisfaisante.

Plus particulièrement, le surfactif anionique, qui constitue un composant essentiel de l'invention, est utilisé à raison de 8 à 20% en poids environ du poids total de la composition, et de préférence entre 9 et 12% en poids. Il est possible d'utiliser un ou plusieurs surfactifs en combinaison, et on utilise de préférence un sulfate d'alkyle ou un éther sulfate d'alkyle dont les propriétés sont bien connues dans le domaine des shampooings.

On peut utiliser par exemple un alkyl sulfate de formule générale $ROSO_3M$, ou un alkyl éther sulfate de formule générale $R(OCH_2CH_2)_nOSO_3M$ dans laquelle R représente un groupe alkyle de 12 à 18 atomes de carbone, M est un cation métallique, un groupe ,$NH_4$ ou un reste aminé, et n est égal à 2 ou 3; M est de préférence un cation de métal-alcalin tel que le sodium ou le potassium. Des surfactifs anioniques particulièrement préférés, entrant dans la formule ci-dessus, sont le lauryl sulfate de sodium, et le lauryl éther sulfate de sodium; d'autres sels peuvent aussi être utilisés, tels que les sels de potassium, d'ammonium et d'éthanolamines. On peut utiliser également d'autres surfactifs anioniques connus tels que des alkyl sulfonates ou des alkyl sulfosuccinates comportant de préférence 12 à 18 atomes de carbone dans la partie alkyle, ou encore des N-acyl sarcosinates de formule $RCONCH_3COOM$ où R est un groupe alkyle de 8 à 18 atomes de carbone et M est un atome d'hydrogène ou un cation métallique.

Le surfactif anionique de formule générale ci-dessus peut être utilisé seul ou en combinaison avec un autre surfactif de la même formule générale, ou avec un autre surfactif anionique différent. Dans ce dernier cas, cet autre surfactif anionique peut être choisi librement parmi les surfactifs anioniques connus, et on peut utiliser par exemple un polypeptidate de métal alcalin tel que le cocopolypeptidate de potassium, un alkyl benzène sulfonate, et par exemple le dodécyl benzène sulfonate de sodium, un alkyl sulfate de diéthanolamine ou de triéthanolamine, etc.

Suivant l'invention, il est particulièrement avantageux d'utiliser en combinaison un sulfate d'alkyle ou un éther sulfate d'alkyle, et plus particulièrement le lauryl sulfate de sodium ou le lauryl éther sulfate de sodium, avec un polypeptidate de métal alcalin, en particulier le cocopolypeptidate de potassium, dans un rapport en poids compris entre 15:1 et 5:1, et de préférence voisin de 10:1.

Le surfactif amphotère qui peut être utilisé dans l'invention constitue un composant secondaire présent en plus faible quantité que le surfactif anionique qui est le composant principal de la base lavante. Il représente moins de 6% en poids environ de la composition de l'invention, et de préférence 0,7 à 2% en poids. Bien entendu, il est possible d'utiliser un mélange de plusieurs surfactifs amphotères, mais leur concentration totale ne doit pas représenter plus de 30% environ de celle des surfactifs anioniques utilisés.

D'une manière générale, il peut être choisi parmi les N-alkyl-β-amino propionates de formule $RNHCH_2CH_2COOM$, ou les N-alkyl-β-amino dipropionates de formule $RN(CH_2CH_2COOM)_2$ dans lesquelles R est un groupe alkyle de 8 à 18 atomes de carbone et M est un atome d'hydrogène ou un cation métallique; des bétaïnes telles que des alkyl-bétaïnes de formule $R(CH_3)_2N^+CH_2COO^-$, des amido-bétaïnes $RCON^+(CH_3)_2CH_2COO^-$, ou des sulfo-bétaïnes de formule $RCON^+(CH_3)_2(CH_2)_3SO_3^-$, dans lesquelles R est un groupe alkyle de 8 à 18 atomes de carbone; des imidazolines de formule

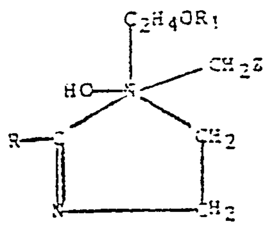

ou

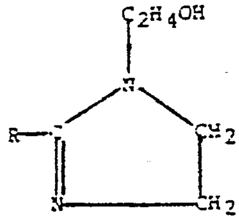

dans lesquelles R est un groupe alkyle de 8 à 18 atomes de carbone, $R_1$ est un atome d'hydrogène, un cation métallique ou un groupe $-CH_2COOM$ où M est un atome d'hydrogène ou un cation métallique, et Z est un groupe $-COOM$ ou $-CH_2COOM$ où M est un atome d'hydrogène ou un cation métallique.

Un surfactif amphotère préféré suivant la présente invention est la cocoamido propyl bétaïne, de préférence en combinaison avec un phosphoamino lipide hydrophile, dans un rapport en poids égal à environ 9:1. On peut par exemple utiliser en combinaison la cocoamido propyl bétaïne et une lécithine modifiée pour la rendre hydrosoluble.

Suivant une forme avantageuse de réalisation de l'invention, le rapport pondéral du surfactif anionique au surfactif amphotère est voisin de 10:1.

Les propriétés de conditionneur de la composition suivant l'invention sont conférées par la combinaison de l'oxyde d'amine et du polymère quanternaire cationique, qui constituent, avec les surfactifs, des composants essentiels. Cette combinaison de composants permet de restaurer les qualités des cheveux, en particulier leur souplesse et leur lustre, et d'améliorer la facilité de coiffage, que les cheveux soient secs ou humides.

Conformément à la présente invention, on utilise un oxyde d'amine présentant un effet conditionneur, comportant dans sa molécule au moins un groupe alkyle linéaire ou ramifié, ou une chaîne alkylène linéaire ou ramifiée, contenant au moins 14 atomes de carbone, et de préférence au moins 16 atomes de carbone.

L'oxyde d'amine est représenté de préférence par la formule générale:

$$\begin{array}{ccc} & R_1 & \\ & | & \\ R_2 - & N & \rightarrow O \\ & | & \\ & R_3 & \end{array}$$

dans laquelle $R_1$ et $R_2$, identiques ou différents, représentent un groupe alkyle inférieur ou hydroxy alkyle inférieur de 1 à 4 atomes de carbone, et $R_3$ représente un groupe alkyle linéaire ou ramifié de 14 à 20 atomes de carbone, ou un groupe de formule R-CONH-R'- dans laquelle R est un groupe alkyle linéaire ou ramifié de 13 à 19 atomes de carbone, et R' une chaîne alkylène divalente de 1 à 4 atomes de carbone, le nombre total d'atomes de carbone étant alors au moins égal à 15. Le groupe alkyle représenté par $R_1$ et $R_2$ peut être un groupe méthyle, éthyle, isopropyle ou n-butyle, mais il est de préférence un groupe méthyle. $R_3$, ou R le cas échéant, doit être un groupe ballast comportant au moins 13 atomes de carbone, et R peut être par exemple un groupe alkyle linéaire à 13 ou 17 atomes de carbone, consituant, avec le groupe -CO- auquel il est rattaché, un groupe myristyle ou stéaryle, respectivement.

Ces oxydes d'amine sont des produits connus et disponibles dans le commerce, généralement préparés par réaction d'une amine tertiaire avec le peroxyde d'hydrogène dans des conditions contrôlées. L'amine tertiaire de départ peut être par exemple une alkyl diméthyl amine, une alkylamidoalkyl diméthyl amine, une alkyl bis-hydroxypropyl amine, ou encore une alkyl morpholine.

Suivant la présente invention, on utilise de préférence un ou plusieurs oxydes d'amine choisis parmi l'oxyde de diméthyl stéaryl amine, l'oxyde de diméthyl cétyl amine, l'oxyde de diméthyl myristyl-cétyl amine, et l'oxyde de suif-amido-propyl diméthyl amine. Ces composés confèrent non seulement des propriétés de conditionneur, mais facilitent aussi la mise en émulsion et la mouillabilité.

La quantité d'oxyde d'amine utilisée dans l'invention représente entre 1 et 3,5% en poids de la composition, et de préférence entre 1 et 3% en poids.

Comme indiqué ci-dessus, les oxydes d'amine utilisés dans l'invention sont des produits connus; par exemple, l'oxyde de diméthyl stéaryl amine a déjà été incorporé dans des formules de shampooing pour le conditionnement du cheveu, ou pour réduire l'irritation que peuvent provoquer les surfactifs. Ils sont également connus comme agents moussants ou stabilisants de la mousse, selon la longueur de la chaîne alkyle de leur molécule. Par contre, ils n'ont pas été utilisés en combinaison avec des polymères quaternaires cationiques en proportions définies, dans une matrice spécifique, pour formuler une composition capable d'agir à la fois comme un shampooing et comme un conditionneur après-shampooing en une même application. Les oxydes d'amine utilisés dans l'invention présentent l'avantage d'être compatibles avec les autres composants et de ne pas réagir avec eux, notamment avec les surfactifs anioniques. De plus, ils

agissent comme composés faiblement cationiques et renforcent l'action de conditionneur des polymères quaternaires cationiques. En combinant dans des proportions déterminées des oxydes d'amine et des polymères quaternaires cationiques, qui ont une substantivité modérée vis-à-vis des cheveux, avec une base de shampooing appropriée, dans la composition de l'invention, suivant les rapports pondéraux indiqués, il est possible d'obtenir d'excellentes propriétés de moussage et un effet de conditionneur tout à fait satisfaisant sans laisser de dépôt excessif des ingrédients conditionneurs sur les cheveux.

Le polymère quaternaire cationique utilisé dans l'invention permet de compléter et d'améliorer les propriétés de conditionneur de la composition tout en étant compatible avec les surfactifs constituant la base lavante. Un ou plusieurs polymères quaternaires cationiques peuvent être utilisés dans la composition de l'invention. Il peut être choisi parmi les polymères quaternaires connus présentant de préférence une faible cationicité. On peut utiliser par exemple certains sels d'ammonium quaternaire dérivés d'acides gras, tels que le quaternium 27, des copolymères d'acrylamide et de chlorure de diméthyl diallyl ammonium tels que le polyquaternium 7, des polymères cellulosiques dérivés d'hydroxyéthyl cellulose et de triéthyl ammonium époxydé tels que le polyquaternium 10, des polymères dérivés de vinyl pyrrolidone, des polymères résultant de la réaction d'un alkyl-sulfate avec un copolymère de vinyl pyrrolidone et de méthacrylate de dialkylamino-éthyle tels que le polyquaternium 11, ou des copolymères d'acrylamide et de chlorure de méthacryloyl alkyl ammonium, etc.

La quantité de polymère quaternaire cationique utilisé dans l'invention est comprise entre 0,2 et 3% environ en poids de la composition, et de préférence entre 0,2 et 1,5%.

Divers additifs habituellement utilisés dans les shampooings et les conditionneurs peuvent être incorporés dans la composition de l'invention, en fonction de leurs propriétés et des résultats recherchés. On peut par exemple ajouter aux composants ci-dessus divers colorants et parfums, des solvants organiques tels que des alcools ou des glycols, des conservateurs tels que des derivés de thiazolinone, et par exemple la méthylchlorothiazolinone, etc.

La composition conforme à la présente invention peut être préparée par les techniques conventionnnelles bien connues.

Les exemples suivants illustrent l'invention sans en limiter la portée.

EXAMPLE 1

Une composition conforme à la présente invention est préparée à partir des composants indiqués au tableau 1 ci-après. Dans ce tableau, tous les pourcentages et parties sont exprimés en poids.

Surfactifs:
Lauryl sulfate de sodium 9,1%
Cocopolypetidate de potassium 1,0%
Cocoamido propyl bétaïne 0,9%
Lécithine modifiée 0,1%

Oxydes d'amine:
Oxyde de diméthyl stéaryl amine 1.3%
Oxyde de diméthyl myristyl-cétyl amine 0,4%

Polymères quaternaires cationiques:
Polyquaternium 7 0,5%
Polyquaternium 10 0,2%

Agent humectant:
Urée 3,0%

Agent séquestrant:
Acide nitrilotriacétique 1,0%

Divers:
Diéthanolamide de coco 1,0%
Monoéthanolamide de coco 0,8%
Undécylènamide MEA 0,1%
Stéarate d'éthylène glycol 1,2%
Amino méthyl propanol 0,6%
Ethyl éther du panthénol 0,3%
Conservateur (Kathon CG) 0,1%
Butyl glycol 1.0%
Colorant, parfum
Eau désionisée qsp 100,0%

Les essais effectués avec la composition ci-dessus, appliqué une seule fois sur les cheveux, avec additon d'eau et moussage, suivi d'un rinçage, ont permis de constater d'excellents résultats.

La facilité de coiffage est nettement améliorée par rapport à un shampooing usuel et est équivalente à celle procurée par les utilisations sucessives d'un shampooing et d'un conditionneur classiques. Des évaluations sensorielles effectuées en laboratoire de coiffure, ainsi que la mesure objective des forces de peignage mesurées par une technique dynamométrique, ont montré une amélioration statistiquement significative du démêlage sur cheveux mouillés et sur cheveux secs.

EXAMPLE 2

On procède comme dans l'exemple 1 en utilisant les composants indiqués ci-après:

Surfactifs:
Lauryl éther sulfate de sodium 11,0%
Cocopolypetidate de potassium 1,2%

Oxydes d'amine:
Oxyde de cétyl-myristyl diméthyl amine 0.5%
Oxyde de suif-amido-propyl diméthyl amine 0,5%
Oxyde de diméthyl stéaryl amine 0,5%

Polymères quaternaires cationiques:
Polyquaternium 11 0,6%
Polyquaternium 10 0,1%

Agent mouillant:
Urée 2,8%

Agent séquestrant:
Acide éthylène diamine tétracétique 0,9%

Divers:
Lauryl monoéthanolamide 1,0%
Lauryl diéthanolamide 0,6%
Stéarate d'éthylène glycol 1,0%
Amino méthyl propanol 0,5%
Ethyl éther du panthénol 0,2%
Conservateur (Kathon CG) 0,1%
Butyl glycol 1.0%
Alcool éthylique infecté 1.0%
Colorant, parfum
Eau désionisée qsp 100,0%

**Revendications**

1. Composition pour le lavage et le conditionnement des cheveux agissant comme shampooing et comme conditionneur, caractérisée en ce que elle est constituée par une solution aqueuse de pH comprise entre 5 et 7 environ, comprenant :

a - 8 à 20% en poids environ d'au moins un surfactif anionique;

b - 1 à 3,5% en poids environ d'au moins un oxyde d'amine présentant un effet de conditionneur, comportant dans sa molécule au moins un groupe alkyle linéaire ou ramifié, ou une chaîne alkylène linéaire ou ramifiée, contenant au moins 14 atomes de carbone;

c - 0,2 à 3% en poids environ d'au moins un polymère quanternaire cationique présentant un effet de conditionneur;

le reste de la composition comprenant des additifs connus.

2. Composition pour le lavage et le conditionnnement des cheveux agissant comme shampooing et comme conditionneur, caractérisée en ce que elle est constituée par une solution aqueuse de pH comprise entre 5,5 et 6,5 environ, comprenant :

a - 9 à 12% en poids environ d'au moins un surfactif anionique;

b - 1 à 3% en poids environ d'au moins un oxyde d'amine présentant un effet de conditionneur, comportant dans sa molécule au moins un groupe alkyle linéaire ou ramifié, ou une chaîne alkylène linéaire ou ramifiée, contenant au moins 14 atomes de carbone;

c - 0,2 à 1,5% en poids environ d'au moins un polymère quaternaire cationique présentant un effet de conditionneur;

d - 0,7 à 2% en poids environ d'au moins un surfactif amphotère;

le reste de la composition comprenant des additifs connus.

3. Composition selon l'une quelconque des revendications 1 et 2, caractérisée en ce qu'elle contient en outre au moins un agent humectant et/ou un agent séquestrant.

4. Composition selon la revendication 3, caractérisée en ce que l'agent humectant est une urée, ou un dérivé, présent à raison de 1 à 5% en poids environ par rapport au poids total de la composition.

5. Composition selon la revendication 3, caractérisée en ce que l'agent séquestrant est l'acide nitrilotriacétique ou l'acide éthylène diaminetétracétique, présent à raison de 0,2 à 2% en poids environ du poids total de la composition.

6. Composition selon la revendication 2, caractérisée en ce que le rapport pondéral du surfactif anionique au surfactif amphotère est voisin de 10:1.

7. Composition selon l'une quelconque des revendications 1 et 2, cartactérisée en ce que le surfactif anionique est un alkyl sulfate ou un alkyl éther sulfate de métal alcalin.

8. Composition selon l'une quelconque des revendications 1 et 2, caractérisée en ce que le surfactif anionique est constitué par le lauryl sulfate de sodium ou le lauryl ether sulfate de sodium en combinaison avec le cocopolypeptidate de potassium, dans un rapport en poids compris entre 15:1 et 5:1.

9. Composition selon la revendication 2, caractérisée en ce que le surfactif amphotère est la cocoamido propyl bétaïne en combinaison avec une lécithine modifiée, dans un rapport en poids égal à environ 9:1.

10. Composition selon l'une quelconque des revendications 1 et 2, caractérisée en ce que l'oxyde d'amine est représenté par la formule générale

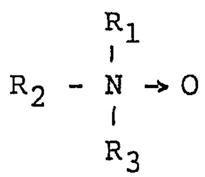

dans laquelle $R_1$ et $R_2$, identiques ou différents, représentent un groupe alkyle inférieur ou hydroxy alkyle inférieur de 1 à 4 atomes de carbone, et $R_3$ représente un groupe alkyl linéaire ou ramifié de 14 à 20 atomes de carbone, ou un groupe de formule R-CONH-R'- dans laquelle R est un groupe alkyle linéaire ou ramifié de 13 à 19 atomes de carbone, et R' une chaîne alkylène divalente de 1 à 4 atomes de carbone, le nombre total d'atomes de carbone étant alors au moins égal à 15.

11. Composition selon la revendication 10, caractérisée en ce que l'oxyde d'amine est choisi parmi l'oxyde de diméthyl stéaryl amine, l'oxyde de diméthyl cétyl amine, l'oxyde de diméthyl myristyl-cétyl amine, et l'oxyde de suif-amido-propyl diméthyl amine.

12. Composition selon l'une quelconque des revendications 1 er 2, caractérisée en ce que le polymère quaternaire cationique est choisi parmi le quaternium 27, le polyquaternium 7, le polyquaternium 10, et le polyquaternium 11.